# EUROPÄISCHE PATENTSCHRIFT

(11) **EP 3 535 019 B1**
(45) Veröffentlichungstag und Bekanntmachung des Hinweises auf die Patenterteilung: **19.08.2020**
(21) Anmeldenummer: 17772353.3
(22) Anmeldetag: 18.09.2017
(51) Int. Cl.: A61N 1/36, A61B 5/0476

(54) **VORRICHTUNG ZUR TRANSKRANIELLEN HIRNSTIMULATION**
DEVICE FOR TRANSCRANIAL BRAIN STIMULATION
DISPOSITIF DE STIMULATION TRANSCRÂNIENNE DU CERVEAU

(30) Priorität: 02.11.2016 DE 102016221478
(43) Veröffentlichungstag der Anmeldung: 11.09.2019
(73) Patentinhaber: Carl von Ossietzky Universität Oldenburg, 26129 Oldenburg (DE)
(72) Erfinder: HERRMANN, Christoph, 26131 Oldenburg (DE)
(74) Vertreter: Fink Numrich Patentanwälte PartmbB
(86) Internationale Anmeldenummer: PCT/EP2017/073393
(87) Internationale Veröffentlichungsnummer: WO 2018/082839

(56) Entgegenhaltungen:
- US-A- 4 858 612
- US-B1- 9 272 118
- US-B1- 9 358 393
- LARS RIECKE ET AL: "4-Hz Transcranial Alternating Current Stimulation Phase Modulates Hearing", BRAIN STIMULATION, Bd. 8, Nr. 4, 1. Juli 2015 (2015-07-01), Seiten 777-783, XP055417996, AMSTERDAM, NL ISSN: 1935-861X, DOI: 10.1016/j.brs.2015.04.004
- Lars Riecke: "Studying Effects of Transcranial Alternating Current Stimulation on Hearing and Auditory Scene Analysis" In: "ADVANCES IN EXPERIMENTAL MEDICINE AND BIOLOGY", 1. Januar 2016 (2016-01-01), Springer, US, XP055417892, ISSN: 0065-2598 Bd. 894, Seiten 371-379, DOI: 10.1007/978-3-319-25474-6_39, das ganze Dokument

## Beschreibung

Die Erfindung betrifft eine Vorrichtung zur transkraniellen Hirnstimulation sowie ein Verfahren und eine Einrichtung zur Kalibrierung dieser Vorrichtung.

Zur Verbesserung der Hörfähigkeit von hörgeschädigten Personen sind nicht-invasive Hörhilfen bekannt, welche ein akustisches Signal durch ein Mikrophon aufzeichnen und anschließend das Schallsignal im Ohr wiedergeben. Nicht-invasive Hörhilfen eignen sich jedoch nicht für Personen, deren Hörfähigkeit sehr stark reduziert ist.

Bei schwer hörgeschädigten Personen werden häufig invasive Hörhilfen verwendet. Im Besonderen kommen dabei Cochlea-Implantate zum Einsatz, welche den Hörnerv der hörgeschädigten Personen elektrisch stimulieren. Implantierte Hörhilfen erzielen zwar gute Resultate, erfordern aber einen chirurgischen Eingriff.

In dem Dokument EP 2 224 987 B1 ist eine Vorrichtung zur Unterdrückung von Tinnitus beschrieben, bei der u.a. auch eine Hirnstimulation dazu genutzt wird, den Tinnitus eines Patienten zu vermindern.

Die Druckschrift LARS RIECKE ET AL: "4-Hz Transcranial Alternating Current Stimulation Phase Modulates Hearing", BRAIN STIMULATION, Bd. 8, Nr. 4, 1. Juli 2015, Seiten 777-783, AMSTERDAM, NL, offenbart eine Vorrichtung zur transkraniellen Hirnstimulation gemäß dem Oberbegriff des Anspruchs 1. Dieses Dokument sowie die weitere Druckschrift Lars Riecke: "Studying Effects of Transcranial Alternating Current Stimulation on Hearing and Auditory Scene Analysis" In: "ADVANCES IN EXPERIMENTAL MEDICINE AND BIOLOGY", 1. Januar 2016, Springer, US, Bd. 894, Seiten 371-379, beschreiben Untersuchungen bezüglich der Auswirkungen einer transkraniellen Hirnstimulation im Bereich des auditorischen Kortex auf die auditorische Wahrnehmung von Menschen.

Aufgabe der Erfindung ist es, eine Vorrichtung zu schaffen, welche mittels transkranieller Hirnstimulation die Hörfähigkeit einer hörgeschädigten Person verbessert.

Diese Aufgabe wird durch die Vorrichtung gemäß Patentanspruch 1 gelöst. Weiterbildungen der Erfindung sind in den abhängigen Ansprüchen definiert.

Die erfindungsgemäße Vorrichtung dient zu transkraniellen Hirnstimulation und umfasst eine akustische Erfassungseinrichtung, wie z.B. ein Mikrofon, mit der akustische Signale als analoge Schallsignale erfasst werden. Die analogen Schallsignale stellen somit die durch die akustische Erfassungseinrichtung erfassten Schalldrücke dar. Ein jeweiliges analoges Schallsignal repräsentiert insbesondere eine vorgegebene Sprachsequenz, wie z.B. einen von einem Menschen ausgesprochenen Satz.

Die Vorrichtung beinhaltet ferner eine Elektrodenanordnung aus mehreren Elektroden, welche in einer Betriebskonfiguration an einem Schädel eines Menschen oder Tieres angeordnet werden können, wobei in der Betriebskonfiguration bei Bestromung der Elektroden ein Stromfluss durch zumindest einen auditorischen Kortex der Hirnrinde im Schädel erzeugt wird. Je nach Ausgestaltung der Vorrichtung kann dabei lediglich der auditorische Kortex in der linken Schädelhälfte oder lediglich der auditorische Kortex in der rechten Schädelhälfte betrachtet werden. In einer bevorzugten Variante wird jedoch sowohl der auditorische Kortex in der linken Schädelhälfte als auch der auditorische Kortex in der rechten Schädelhälfte bei der Anordnung der Elektroden berücksichtigt. Auf diese Weise kann die Hörfähigkeit beider Ohren des entsprechenden Menschen oder Tieres verbessert werden.

Die erfindungsgemäße Vorrichtung umfasst ferner eine Signalkonvertierungseinrichtung. Diese Einrichtung ist dazu konfiguriert, ein jeweiliges analoges Schallsignal in ein erstes digitales Signal zu wandeln, das in seinem Verlauf dem jeweiligen analogen Schallsignal entspricht. Unter dem Begriff des Verlaufs ist hier und im Folgenden der zeitliche Verlauf zu verstehen. Ferner ist die obige Formulierung, wonach ein Signal in seinem Verlauf einem anderen Signal entspricht, hier und im Folgenden derart zu verstehen, dass beide Signale bis auf einen Skalierungsfaktor miteinander übereinstimmen.

Die Signalkonvertierungseinrichtung ist ferner derart ausgestaltet, dass sie in ihrem Betrieb aus dem ersten digitalen Signal ein zweites digitales Signal ermittelt, indem aus dem ersten digitalen Signal die Hüllkurve extrahiert wird und die Hüllkurve um einen vorbestimmten Zeitbetrag größer Null in die Zukunft verschoben wird bzw. ggf. auch unverschoben bleibt. Die Verschiebung in die Zukunft entspricht einer Verschiebung der Hüllkurve entlang der Zeitachse hin zu einem späteren Zeitpunkt. Mit anderen Worten kann diese Verschiebung als eine zeitliche Verzögerung der Hüllkurve und damit des weiter unten beschriebenen gewandelten Stroms aufgefasst werden.

Der Begriff der Hüllkurve ist dem Fachmann geläufig und entsprechende Verfahren zu deren Extraktion sind an sich bekannt. Der Begriff der Hüllkurve beschreibt die zeitliche Einhüllende, welche durch lokale Maxima der Absolutwerte des entsprechenden Signals verläuft, für das die Hüllkurve extrahiert wird. Dabei werden in der Regel nicht alle lokalen Maxima der Absolutwerte betrachtet, sondern die zeitliche Einhüllende wird in einem vorbestimmten Frequenzbereich des Signals bestimmt. Hier und im Folgenden fällt unter dem Begriff der Hüllkurve somit die zeitliche Einhüllende und ggf. auch die zeitliche Einhüllende, welche einer Korrektur unterzogen wurde. Eine solche Korrektur kann beispielsweise darin bestehen, dass Werte der (unkorrigierten) zeitlichen Einhüllenden, welche einen vorbestimmten prozentualen Wert (z.B. 25%) des absoluten Maximums der zeitlichen Einhüllenden überschreiten, auf das absolute Maximum gesetzt werden. Hierdurch wird eine Verzerrung der zeitlichen Einhüllenden erreicht.

Die Signalkonvertierungseinrichtung der erfindungsgemäßen Vorrichtung ist ferner derart ausgestaltet, dass sie das obige zweite digitale Signal in einen elektrischen Strom wandelt, der in seinem Verlauf dem zweiten digitalen Signal entspricht, und die Elektrodenanordnung in der Betriebskonfiguration mit diesem Strom bestromt.

Der Erfindung liegt die Erkenntnis zugrunde, dass mittels einer transkraniellen Hirnstimulation, welche der Hüllkurve eines entsprechenden Schallsignals entspricht, eine deutliche Verbesserung der Hörfähigkeit einer Person erreicht werden kann. Die Anbringung der erfindungsgemäßen Vorrichtung erfordert dabei keinen chirurgischen Eingriff, wie dies bei invasiven Hörhilfen der Fall ist. Es müssen lediglich Elektroden an entsprechende Areale am Schädel der Person angebracht werden.

In einer besonders bevorzugten Variante besteht die Elektrodenanordnung aus einem Paar von Elektroden für den auditorischen Kortex in der linken Schädelhälfte und/oder einem Paar von Elektroden für den auditorischen Kortex in der rechten Schädelhälfte. In der Vorrichtung werden somit maximal zwei Elektrodenpaare verwendet, wodurch die Anbringung der Vorrichtung vereinfacht wird und deren Tragekomfort verbessert wird.

In einer weiteren bevorzugten Ausführungsform liegt der vorbestimmte Zeitbetrag, um den die Hüllkurve in die Zukunft verschoben wird, zwischen 50 ms und 150 ms, vorzugsweise bei 100 ms. Es konnte experimentell gezeigt werden, dass diese Werte für die meisten Patienten die beste Verbesserung der Hörfähigkeit liefern.

In einer weiteren bevorzugten Ausführungsform wird im Rahmen der Extraktion der Hüllkurve eine Hilbert-Transformation des ersten digitalen Signals durchgeführt. Diese Hilbert-Transformation ist an sich bekannt und liefert für jeden zeitlichen Signalwert des ersten digitalen Signals eine komplexe Zahl. Die zeitliche Abfolge der Beträge dieser komplexen Zahlen entspricht der (ungefilterten) Hüllkurve des ersten digitalen Signals.

In einer weiteren bevorzugten Ausgestaltung ist die Signalkonvertierungseinrichtung derart ausgestaltet, dass die Extraktion der Hüllkurve eine Bandpassfilterung des ersten digitalen Signals nach dessen Hilbert-Transformation in einem vorbestimmten Frequenzbereich umfasst. Insbesondere ist dieser Frequenzbereich das Band zwischen 0 Hz und 10 Hz oder ein Teilabschnitt aus diesem Band, wie z.B. das Band zwischen 4 Hz und 8 Hz. Diese Frequenzbereiche spiegeln sehr gut das Frequenzspektrum einzelner Silben in Sprachsignalen wider.

In einer weiteren bevorzugten Ausführungsform der erfindungsgemäßen Vorrichtung umfasst die Signalkonvertierungseinrichtung eine Signalverarbeitungseinheit und eine Stimulationseinheit. Diese sind derart ausgestaltet, dass die Signalverarbeitungseinheit das jeweilige Schallsignal von der akustischen Erfassungseinrichtung empfängt und hieraus das zweite digitale Signal ermittelt, wobei das zweite digitale Signal durch die Signalverarbeitungseinheit zunächst in eine elektrische Spannung gewandelt wird, die in ihrem Verlauf dem zweiten digitalen Signal entspricht und welche an die Stimulationseinheit ausgegeben wird. Die Stimulationseinheit wandelt diese Spannung in den oben beschriebenen elektrischen Strom, mit dem die Elektrodenanordnung bestromt wird.

In einer weiteren bevorzugten Variante der erfindungsgemäßen Vorrichtung ist der Strom zur Bestromung der Elektrodenanordnung derart festgelegt, dass sein Maximalwert zwischen 0,5 mA und 2 mA, insbesondere bei 2 mA oder weniger und vorzugsweise bei 1 mA, liegt. Auf diese Weise wird sichergestellt, dass der Strom unterhalb der Wahrnehmungsschwelle des Patienten liegt.

In einer weiteren, bevorzugten Ausführungsform ist die erfindungsgemäße Vorrichtung (als ganzes) durch den Menschen oder das Tier, an dessen Schädel die Elektroden der Elektrodenanordnung in der Betriebskonfiguration angebracht sind, tragbar. Im Besonderen ist sie (als ganzes) am Schädel des Menschen oder Tiers befestigbar. Auf diese Weise wird gewährleistet, dass die Vorrichtung durch den Patienten mitgeführt werden kann.

In einer weiteren bevorzugten Ausführungsform umfasst die erfindungsgemäße Vorrichtung ferner eine akustische Ausgabeeinrichtung zur Ausgabe von akustischen Signalen basierend auf den analogen Schallsignalen im Bereich eines oder beider Ohren des Menschen oder Tiers, an dessen Schädel die Elektroden der Elektrodenanordnung in der Betriebskonfiguration angebracht sind. Diese Ausgabe erfolgt vorzugsweise unmittelbar nach Erfassung der akustischen Signale durch die akustische Erfassungseinrichtung. Durch diese zusätzliche akustische Ausgabe kann die Hörfähigkeit des Patienten weiter verbessert werden. Die basierend auf den analogen Schallsignalen ausgegebenen akustischen Signale stimmen dabei im Wesentlichen mit den ursprünglich erfassten akustischen Signalen überein, können jedoch ggf. einer geeigneten Nachverarbeitung zur Signalverbesserung unterzogen werden. Zum Beispiel kann Signalrauschen entfernt oder unterdrückt werden.

Neben der oben beschriebenen erfindungsgemäßen Vorrichtung umfasst die Erfindung ferner ein Verfahren zu deren Kalibrierung. Im Rahmen dieses Kalibrierverfahrens wird bei Vorliegen eines akustischen Signals mittels einer Elektrodenanordnung aus mehreren Elektroden ein Elektroenzephalogramm in der Form eines zeitlichen Spannungsverlaufs im Bereich zumindest eines auditorischen Kortex der Hirnrinde im Schädel eines Menschen oder Tieres erfasst, für welchen bzw. für welches die erfindungsgemäße Vorrichtung zu verwenden ist. Hierzu werden die Elektroden der Elektrodenanordnung an geeigneten Positionen am Schädel angebracht. Insbesondere können diese Positionen der obigen Betriebskonfiguration entsprechen. Vorzugsweise besteht die Elektrodenanordnung aus einem Paar von Elektroden für den auditorischen Cortex in der linken Schädelhälfte und/oder einem Paar von Elektroden für den auditorischen Cortex in der rechten Schädelhälfte. Die Elektrodenanordnung kann ggf. die Elektrodenanordnung der zu kalibrierenden Vorrichtung sein.

Mit analogen Methoden wie in der erfindungsgemäßen Vorrichtung wird die (digitale bzw. digitalisierte) Hüllkurve des Elektroenzephalogramms extrahiert und der Zeitversatz mit größter Übereinstimmung zwischen der durch die zu kalibrierende Vorrichtung extrahierten Hüllkurve, welche auf dem vorliegenden akustischen Signal basiert und parallel zur Erfassung des Elektroenzephalogramms berechnet wird, und der Hüllkurve des Elektroenzephalogramms mittels Kreuzkorrelation ermittelt. Es wird somit in an sich bekannter Weise der maximale Korrelationswert entsprechend der Kreuzkorrelation der beiden Hüllkurven bestimmt. Dieser Zeitversatz wird anschließend als vorbestimmter Zeitbetrag in der zu kalibrierenden Vorrichtung hinterlegt und dann von dieser verwendet. Bei der Durchführung der Kalibrierung wird durch die Vorrichtung zur transkraniellen Hirnstimulation vorzugsweise lediglich die Hüllkurve extrahiert, ohne dass der Elektrodenanordnung der Vorrichtung elektrischer Strom zugeführt wird.

Mit dem soeben beschriebenen Kalibrierverfahren wird eine patientenspezifische Anpassung der Vorrichtung an den Zeitversatz zwischen dem Auftreten von akustischen Signalen und deren Verarbeitung im Gehirn erreicht. Hierdurch wird die erfindungsgemäße Vorrichtung optimal auf den entsprechenden Träger eingestellt.

Neben dem oben beschriebenen Kalibrierverfahren umfasst die Erfindung eine Einrichtung zur Kalibrierung, die zur Durchführung dieses Kalibrierverfahrens konfiguriert ist. Mit anderen Worten umfasst diese Einrichtung ein Mittel zur Erfassung eines Enzephalogramms sowie ein Mittel zur Extraktion der Hüllkurve des Enzephalogramms und zum Ermitteln des Zeitversatzes mittels Kreuzkorrelation sowie ein Mittel, um den entsprechenden Zeitversatz an die zu kalibrierende Vorrichtung zu übertragen.

Die soeben beschriebene Kalibriereinrichtung kann eine Komponente sein, welche von der zu kalibrierenden Vorrichtung separiert ist. Nichtsdestotrotz ist es auch möglich, dass die Kalibriereinrichtung Bestandteil der erfindungsgemäßen Vorrichtung zur transkraniellen Hirnstimulation ist.

Ausführungsbeispiele der Erfindung werden nachfolgend anhand der beigefügten Figuren detailliert beschrieben.

Es zeigen:
- Fig. 1: eine schematische Darstellung einer Ausführungsform der erfindungsgemäßen Vorrichtung zur transkraniellen Hirnstimulation; und
- Fig. 2: eine schematische Darstellung eines mittels der Vorrichtung der Fig. 1 erfassten Schallsignals und der daraus extrahierten Hüllkurve.

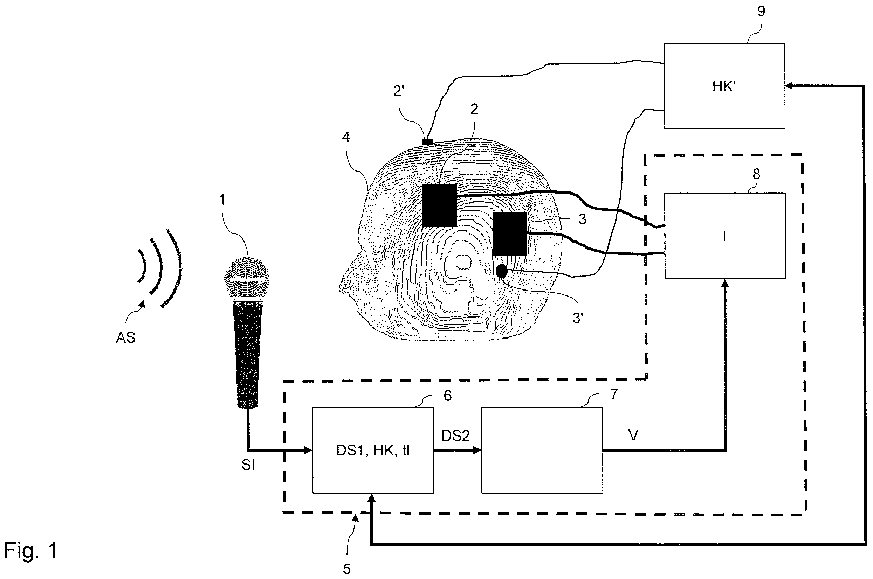

Fig. 1 zeigt in schematischer Darstellung eine Ausführungsform einer erfindungsgemäßen Vorrichtung zur transkraniellen Hirnstimulation. Die Vorrichtung dient dabei dazu, die Hörfähigkeit einer hörgeschädigten Person über eine geeignete Stimulation der Hirnrinde mittels elektrischen Stroms zu verbessern. In diesem Sinne stellt die Vorrichtung eine Hörhilfe dar. Die nachfolgend beschriebenen Komponenten der Vorrichtung sind vorzugsweise derart ausgestaltet, dass sie tragbar sind und durch die hörgeschädigte Person mitgeführt werden können.

Die Vorrichtung der Fig.1 umfasst ein Mikrofon 1, welches vorzugsweise in miniaturisierter Form ausgeführt ist und im Bereich eines oder beider Ohren der hörgeschädigten Person angebracht ist. Über das Mikrofon 1 werden akustische Signale erfasst, welche in Fig. 1 mit AS bezeichnet sind und durch drei konzentrische Kreissegmente angedeutet werden. Die mit dem Mikrofon 1 erfassten akustischen Signale stellen analoge Signale dar, welche in Fig. 1 als Schallsignale SI bezeichnet sind. Ein einzelnes erfasstes Schallsignal SI stellt dabei vorzugsweise eine zusammenhängend gesprochene Sprachsequenz dar, die beispielsweise eine mit der hörgeschädigten Person kommunizierende weitere Person ausspricht. Insbesondere kann es sich bei der Sprachsequenz um einen gesprochenen Satz handeln. Die Schallsignale SI werden einer Signalkonvertierungseinrichtung 5 zugeführt. Die Komponenten dieser Einrichtung sind vorzugsweise in einem gemeinsamen Gehäuse untergebracht, das beispielsweise am oder hinter dem Ohr der hörgeschädigten Person befestigt sein kann.

Am Schädel der hörgeschädigten Person, der in Fig. 1 mit Bezugszeichen 4 bezeichnet ist, sind oberhalb des linken Ohrs zwei Elektroden 2 und 3 befestigt. Die Elektroden sind dabei derart positioniert, dass ein zwischen diesen Elektroden fließender Strom, der durch den weiter unten beschriebenen transkranielle Elektrodenstimulator 8 erzeugt wird, den auditorischen Kortex in der linken Schädelhälfte stimuliert. Der auditorische Kortex der linken Hirnhälfte liegt dabei im 10-20 EEG System etwa unter der Elektrodenposition T3, wohingegen der Kortex der rechten Hirnhälfte etwa unter der Elektrodenposition T4 liegt. Um einen Stromfluss durch den Kortex der linken Hirnhälfte zu erreichen, kann die Elektrode 2 beispielsweise an der Position F3 und die Elektrode 3 beispielsweise an der Position T5 im 10-20 EEG System angeordnet sein. Die Elektroden 2 und 3 sind über elektrische Kabel an den Elektrodenstimulator 8 angeschlossen.

Vorzugsweise sind auch auf der rechten Schädelhälfte zwei Elektroden positioniert, über welche ein Stromfluss durch den rechten auditorischen Kortex mittels des transkraniellen Elektrodenstimulators 8 ermöglicht wird. Eine dieser Elektroden kann beispielsweise an der Position F4 und die andere dieser Elektroden an der Position T6 im 10-20 EGG System angeordnet sein. Durch die Verwendung von Elektroden auf beiden Schädelhälften wird die Hörfähigkeit beider Ohren der hörgeschädigten Person verbessert. Liegt ein Hörschaden nur für eines der Ohren vor, wird nur ein Elektrodenpaar für den auditorischen Kortex des geschädigten Ohrs verwendet. Im Folgenden wird die Vorrichtung nur anhand der Stimulation der Elektroden 2 und 3 beschrieben, wobei die Stimulation der Elektroden auf der anderen Schädelhälfte analog abläuft.

Das mit dem Mikrofon 1 erfasste Schallsignal SI wird einem Signalprozessor 6 innerhalb der Schallkonvertierungseinrichtung 5 zugeführt. Der Signalprozessor 6 sowie der weiter unten beschriebene Digital-Analog-Wandler 7 können beispielsweise in einer gemeinsamen integrierten Schaltung implementiert sein. Das Signal SI wird im Signalprozessor vorverstärkt und anschließend digitalisiert, wodurch ein erstes digitales Signal DS1 erhalten wird. Mittels des Signalprozessors 6 wird in an sich bekannter Weise aus dem ersten digitalen Signal DS1 die Hüllkurve HK (d. h die zeitliche Einhüllende) bestimmt. In der hier beschriebenen Ausführungsform wird die Hüllkurve in einem vorbestimmten Frequenzbereich des ersten digitalen Signals DS1 extrahiert, so dass hochfrequente Schwingungen im Schallsignal nicht mehr in der Hüllkurve enthalten sind. Im Detail wird im Rahmen der Hüllkurvenextraktion auf das digitale Signal DS1 die an sich bekannte Hilbert-Transformation angewendet, welche komplexe Werte liefert, deren Beträge die ungefilterte Hüllkurve darstellen. Anschließend wird diese Kurve einer Tiefpassfilterung in dem Frequenzbereich zwischen 0 Hz und 10 Hz unterzogen, was dem für das Hören relevanten Frequenzbereich einzelner Silben entspricht. Die gefilterte Kurve stellt in der hier beschriebenen Ausführungsform die Hüllkurve HK dar.

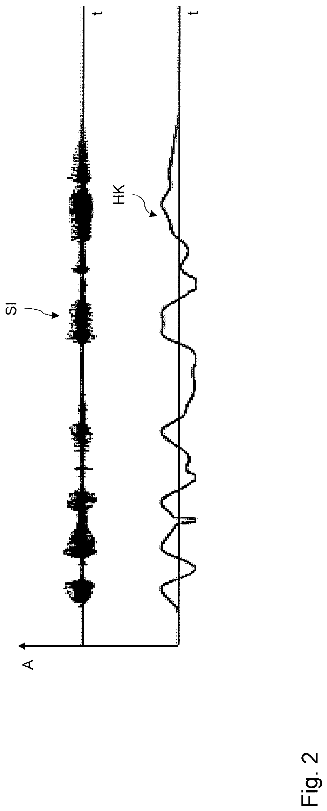

Fig. 2 zeigt ein Diagramm, welches beispielhaft die Extraktion einer Hüllkurve mittels der Vorrichtung aus Fig. 1 wiedergibt. Entlang der Abszisse des Diagramms ist die Zeit t und entlang der Koordinate die Amplitude A wiedergegeben. Das erfasste analoge Schallsignal SI ist im oberen Teil des Diagramms dargestellt. Aus diesem Signal ergibt sich die Hüllkurve HK, die im unteren Teil des Diagramms wiedergegeben ist. Die Hüllkurve ist dabei eine mittelwertfreie Kurve, d. h. die Werte der Hüllkurve geben die Abweichung gegenüber dem Signalmittelwert wieder.

In einem nächsten Schritt wird die Hüllkurve HK in der Signalverarbeitungseinheit 6 um einen vorbestimmten Zeitbetrag tl in die Zukunft verschoben. Der Zeitbetrag spiegelt dabei den Versatz zwischen dem Auftreten des akustischen Signals AS und dem Zeitpunkt der Signalverarbeitung im Gehirn wieder. Der Wert des Zeitbetrags kann vorab durch Experimente geeignet bestimmt worden sein und beispielsweise auf 100 ms gesetzt sein. In einer besonders bevorzugten Variante wird dieser Zeitbetrag im Rahmen eines Kalibriervorgangs der hier beschriebenen Vorrichtung bestimmt, wobei dieser Kalibriervorgang weiter unten näher erläutert wird.

Die um den Zeitbetrag tl verschobene Hüllkurve stellt ein zweites digitales Signal DS2 dar, welches anschließend dem Digital-Analog-Wandler 7 innerhalb der Signalkonvertierungseinrichtung 5 zugeführt wird. Dieser Digital-Analog-Wandler liefert eine elektrische Spannung V, deren zeitlicher Verlauf dem zweiten digitalen Signal DS2 entspricht. Die Spannung V wird schließlich dem transkraniellen Elektrodenstimulator 8 zugeführt, der die Spannung in einen elektrischen Strom wandelt, dessen zeitlicher Verlauf der Spannung V entspricht. Diesen Strom führt der Stimulator 8 den Elektroden 2 und 3 zu. Der Stimulator stellt dabei sicher, dass auch bei einer Veränderung der Impedanz am Schädel der gewünschte Strom durch den auditorischen Kortex zwischen den Elektroden 2 und 3 fließt. Mittels der Elektrodenstimulation entsprechend der Hüllkurve des Schallsignals kann eine deutliche Verbesserung der Hörfähigkeit erreicht werden, insbesondere wenn in dem ursprünglichen akustischen Signal ein hoher Geräuschpegel enthalten ist.

Um den oben beschriebenen Zeitbetrag tl spezifisch an die hörgeschädigte Person zu adaptieren, wird in einer bevorzugten Ausführungsform eine Kalibrierung der erfindungsgemäßen Vorrichtung vorgenommen, wobei hierfür die schematisch angedeutete Kalibriervorrichtung 9 verwendet wird. An diese Kalibriervorrichtung sind zwei Elektroden 2' und 3' angeschlossen, welche ähnlich wie die Elektroden 2 und 3 derart am Schädel 4 angeordnet werden, dass sie den auditorischen Kortex am linken Ohr überbrücken.

Im Rahmen des Kalibriervorgangs wird ein vorgegebenes akustisches Signal erzeugt. Dieses Signal wird wie vorstehend beschrieben durch die Signalkonvertierungseinrichtung 5 verarbeitet, ohne dass jedoch der Strom I durch den Elektrodenstimulator 8 erzeugt wird. Gleichzeitig wird für das ankommende akustische Signal das Elektroenzephalogramm, d.h. die auftretenden Spannungspulse zwischen den Elektroden 2' und 3', durch die Kalibriervorrichtung 9 bestimmt. In Analogie zur oben beschriebenen Extraktion der Hüllkurve HK extrahiert die Vorrichtung 9 aus diesem Elektroenzephalogramm die Hüllkurve HK' der Spannungssignale.

Die Kalibriervorrichtung 9 kann über eine geeignete Schnittstelle mit dem Signalprozessor 6 der zu kalibrierenden Vorrichtung kommunizieren, wie durch einen Doppelpfeil in Fig. 1 angedeutet ist. Über diese Schnittstelle liest die Vorrichtung 9 die extrahierte Hüllkurve HK aus und ermittelt mittels Kreuzkorrelation den Zeitversatz mit der größten Übereinstimmung zwischen der Hüllkurve HK und der Hüllkurve HK'. Es wird somit der Zeitversatz mit dem Maximalwert der Kreuzkorrelationsfunktion der beiden Hüllkurven bestimmt. Dieser Zeitversatz gibt die personenspezifische Zeitverschiebung zwischen dem ankommenden akustischen Signal und dessen Verarbeitung im Gehirn wieder. Der ermittelte Zeitversatz wird anschließend von der Vorrichtung 9 über die oben beschriebene Schnittstelle an den Signalprozessor 6 übermittelt, welcher diesen Zeitversatz als vorbestimmten Zeitbetrag tl speichert und im Betrieb zur Verschiebung der Hüllkurve nutzt. In diesem Sinne stellt der übermittelte Zeitversatz einen kalibrierten Wert für die Vorrichtung zur transkraniellen Hirnstimulation dar.

Die im Vorangegangenen beschriebene Kalibriervorrichtung wurde als separate Komponente beschrieben, die nicht Bestandteil der Vorrichtung zur transkraniellen Hirnstimulation ist. Gegebenenfalls besteht jedoch auch die Möglichkeit, dass die entsprechende Funktion der Kalibrierung in der Vorrichtung zur transkraniellen Hirnstimulation integriert ist.

Die im Vorangegangenen beschriebenen Ausführungsformen der Erfindung weisen eine Reihe von Vorteilen auf. Insbesondere wird eine einfach aufgebaute nicht-invasive Hörhilfe geschaffen, die mittels Hirnstimulation des auditorischen Kortex die Hörfähigkeit von hörgeschädigten Personen verbessert. Die Hörhilfe erfordert im Gegensatz zu Cochlea-Implantaten keinen chirurgischen Eingriff, sondern sie kann auf einfache Weise durch Anbringung von Elektroden am Schädel in Betrieb genommen werden. Mit der erfindungsgemäßen Hörhilfe wird dabei insbesondere eine Verbesserung der Hörfähigkeit für Sprachsignale erreicht, welche in einem starken Hintergrundrauschen eingebettet sind.

## Patentansprüche

1. Vorrichtung zur transkraniellen Hirnstimulation, umfassend:
- eine Elektrodenanordnung aus mehreren Elektroden (2, 3), welche in einer Betriebskonfiguration an einem Schädel (4) eines Menschen oder Tiers angeordnet werden können, wobei in der Betriebskonfiguration bei Bestromung der Elektroden (2, 3) ein Stromfluss durch zumindest einen auditorischen Kortex der Hirnrinde im Schädel (4) erzeugt wird;
**dadurch gekennzeichnet, dass** die Vorrichtung umfasst:
- eine akustische Erfassungseinrichtung (1) zur Erfassung von akustischen Signalen (AS) als analoge Schallsignale (SI);
- eine Signalkonvertierungseinrichtung (5), welche dazu konfiguriert ist,
i) ein jeweiliges analoges Schallsignal (SI) in ein erstes digitales Signal (DS1) zu wandeln, das in seinem Verlauf dem jeweiligen analogen Schallsignal (SI) entspricht, und aus dem ersten digitalen Signal (DS1) ein zweites digitales Signal (DS2) zu ermitteln, indem aus dem ersten digitalen Signal (DS1) die Hüllkurve (HK) extrahiert wird und die Hüllkurve (HK) um einen vorbestimmten Zeitbetrag (tl) größer Null in die Zukunft verschoben wird oder unverschoben bleibt;
ii) das zweite digitale Signal (DS2) in einen Strom (I) zu wandeln, der in seinem Verlauf dem zweiten digitalen Signal (DS2) entspricht, und die Elektrodenanordnung in der Betriebskonfiguration mit diesem Strom (I) zu bestromen.

2. Vorrichtung nach Anspruch 1, **dadurch gekennzeichnet, dass** die Elektrodenanordnung aus einem Paar von Elektroden (2, 3) für den auditorischen Cortex auf der linken Schädelhälfte und/oder einem Paar von Elektroden für den auditorischen Cortex in der rechten Schädelhälfte besteht.

3. Vorrichtung nach Anspruch 1 oder 2, **dadurch gekennzeichnet**, das der vorbestimmte Zeitbetrag (tl) zwischen 50 ms und 150 ms und vorzugsweise bei 100 ms liegt.

4. Vorrichtung nach einem der vorhergehenden Ansprüche, **dadurch gekennzeichnet, dass** die Signalkonvertierungseinrichtung (5) derart ausgestaltet ist, dass im Rahmen der Extraktion der Hüllkurve (HK) eine Hilbert-Transformation des ersten digitalen Signals (DS1) durchgeführt wird.

5. Vorrichtung nach Anspruch 4, **dadurch gekennzeichnet, dass** die Signalkonvertierungseinrichtung (5) derart ausgestaltet ist, dass die Extraktion der Hüllkurve (HK) eine Bandpassfilterung des ersten digitalen Signals (DS1) nach dessen Hilbert-Transformation in einem vorbestimmten Frequenzbereich umfasst, wobei der vorbestimmte Frequenzbereich vorzugsweise das Band zwischen 0 und 10 Hz oder ein Teilabschnitt aus diesem Band ist.

6. Vorrichtung nach einem der vorhergehenden Ansprüche, **dadurch gekennzeichnet, dass** die Signalkonvertierungseinrichtung (5) eine Signalverarbeitungseinheit (6, 7) und eine Stimulationseinheit (8) umfasst, welche derart ausgestaltet sind, dass die Signalverarbeitungseinheit (6, 7) das jeweilige analoge Schallsignal (SI) von der akustischen Erfassungseinrichtung (1) empfängt und hieraus das zweite digitale Signal (DS2) ermittelt, wobei das zweite digitale Signal (DS2) durch die Signalverarbeitungseinheit (6, 7) zunächst in eine Spannung (V) gewandelt wird, die in ihrem Verlauf dem zweiten digitalen Signal (DS2) entspricht und welche an die Stimulationseinheit (8) ausgegeben wird, welche die Spannung (V) in den Strom (I) zur Bestromung der Elektrodenanordnung wandelt.

7. Vorrichtung nach einem der vorhergehenden Ansprüche, **dadurch gekennzeichnet, dass** der Strom (I) derart festgelegt ist, dass sein Maximalwert zwischen 0,5 mA und 5 mA, insbesondere bei 2mA oder weniger und vorzugsweise bei 1 mA liegt.

8. Vorrichtung nach einem der vorhergehenden Ansprüche, **dadurch gekennzeichnet**, das die Vorrichtung durch den Menschen oder das Tier, an dessen Schädel (4) die Elektroden (2,3) oder Elektrodenanordnung in der Betriebskonfiguration angebracht sind, tragbar ist und insbesondere am Schädel (4) des Menschen oder Tiers befestigbar ist.

9. Vorrichtung nach einem der vorhergehenden Ansprüche, **dadurch gekennzeichnet, dass** die Vorrichtung eine akustische Ausgabeeinrichtung umfasst, um akustische Signale basierend auf den analogen Schallsignalen im Bereich eines oder beider Ohren des Menschen oder Tiers auszugeben, an dessen Schädel (4) die Elektroden (2,3) oder Elektrodenanordnung in der Betriebskonfiguration angebracht sind.

10. Verfahren zur Kalibrierung einer Vorrichtung nach einem der vorhergehenden Ansprüche, bei dem
- bei Vorliegen eines akustischen Signals (AS) mittels einer Elektrodenanordnung aus mehreren Elektroden (2', 3') ein Elektroenzephalogramm in der Form eines zeitlichen Spannungsverlaufs im Bereich zumindest eines auditorischen Kortex der Hirnrinde im Schädel (4) eines Menschen oder Tiers erfasst wird, für welchen oder welches die Vorrichtung zur transkraniellen Hirnstimulation zu verwenden ist;
- die Hüllkurve (HK') des Elektroenzephalogramms extrahiert wird und der Zeitversatz mit größter Übereinstimmung zwischen der durch die Vorrichtung zur transkraniellen Hirnstimulation extrahierten Hüllkurve (HK), welche auf dem vorliegenden akustischen Signal (AS) basiert, und der Hüllkurve (HK') des Elektroenzephalogramms mittels Kreuzkorrelation ermittelt wird;
- der Zeitversatz als vorbestimmter Zeitbetrag (tl) in der Vorrichtung zur transkraniellen Hirnstimulation hinterlegt wird.

11. Einrichtung zur Kalibrierung einer Vorrichtung nach einem der Ansprüche 1 bis 9, **dadurch gekennzeichnet, dass** die Einrichtung zur Durchführung des Verfahrens nach Anspruch 10 konfiguriert ist.

12. Vorrichtung zur transkraniellen Hirnstimulation nach einem der Ansprüche 1 bis 9, **dadurch gekennzeichnet, dass** die Einrichtung zur Kalibrierung nach Anspruch 11 Bestandteil der Vorrichtung ist.

## Claims

1. An apparatus for transcranial brain stimulation comprising:
- an electrode arrangement comprising a plurality of electrodes (2, 3) which can be arranged in an operating configuration on a skull (4) of a human being or animal, wherein in the operating configuration during energization of the electrodes (2, 3) a current flow is generated through at least one auditory cortex of the cerebral cortex in the skull (4);
**characterized in that** the apparatus comprises:
- an acoustic detection device (1) for detecting acoustic signals (AS) as analog sound signals (SI);
- a signal conversion device (5) configured to
i) convert a respective analog sound signal (SI) into a first digital signal (DS1), the course of which corresponds to the respective analog sound signal (SI), and to determine a second digital signal (DS2) from the first digital signal (DS1) by extracting the envelope (HK) from the first digital signal (DS1) and shifting the envelope (HK) by a predetermined amount of time (tl) greater than zero into the future or leaving it unshifted;
ii) convert the second digital signal (DS2) into a current (I), the course of which corresponds to the second digital signal (DS2), and to energize the electrode arrangement in the operating configuration with this current (I).

2. The apparatus according to claim 1, **characterized in that** the electrode arrangement consists of a pair of electrodes (2, 3) for the auditory cortex in the left half of the skull and/or a pair of electrodes for the auditory cortex in the right half of the skull.

3. The apparatus according to claim 1 or 2, **characterized in that** the predetermined amount of time (tl) is between 50 ms and 150 ms, and is preferably 100 ms.

4. The apparatus according to one of the preceding claims, **characterized in that** the signal conversion device (5) is configured such that a Hilbert transformation of the first digital signal (DS1) is carried out during the extraction of the envelope (HK).

5. The apparatus according to claim 4, **characterized in that** the signal conversion device (5) is configured such that the extraction of the envelope (HK) comprises a bandpass filtering of the first digital signal (DS1) subsequent to its Hilbert transformation in a predetermined frequency range, the predetermined frequency range preferably being the band between 0 and 10 Hz or a section of this band.

6. The apparatus according to one of the preceding claims, **characterized in that** the signal conversion device (5) comprises a signal processing unit (6, 7) and a stimulation unit (8) which are configured such that the signal processing unit (6, 7) receives the respective analog sound signal (SI) from the acoustic detection device (1) and determines the second digital signal (DS2) therefrom, wherein the second digital signal (DS2) is firstly converted by the signal processing unit (6, 7) into a voltage (V), the course of which corresponds to the second digital signal (DS2) and which is output to the stimulation unit (8) which converts the voltage (V) into the current (I) for energizing the electrode arrangement.

7. The apparatus according to one of the preceding claims, **characterized in that** the current (I) is set such that its maximum value is between 0.5 mA and 5 mA, in particular at 2 mA or less, and is preferably 1 mA.

8. The apparatus according to one of the preceding claims, **characterized in that** the apparatus is wearable by the human being or animal to whose skull (4) the electrodes (2, 3) or the electrode arrangement are attached in the operational configuration, and is attachable in particular to the skull (4) of the human being or animal.

9. The apparatus according to one of the preceding claims, **characterized in that** the device comprises an acoustic output device for outputting acoustic signals based on the analog sound signals in the region of one ear or both ears of the human being or animal to whose skull (4) the electrodes (2, 3) or the electrode arrangement are attached in the operating configuration.

10. A method for calibrating an apparatus according to one of the preceding claims, wherein
- in the presence of an acoustic signal (AS), an electroencephalogram in the form of a temporal voltage curve is detected in the region of at least one auditory cortex of the cerebral cortex in the skull (4) of a human or animal, for which the apparatus for transcranial brain stimulation is to be used, by means of an electrode arrangement comprising a plurality of electrodes (2', 3');
- the envelope (HK') of the electroencephalogram is extracted and the time offset having the greatest correspondence between the envelope (HK) which is extracted by the apparatus for transcranial brain stimulation and which is based on the present acoustic signal (AS), and the envelope (HK') of the electroencephalogram is determined by cross correlation;
- the time offset is stored as a predetermined time amount (tl) in the apparatus for transcranial brain stimulation.

11. A device for calibrating an apparatus according to one of claims 1 to 9, **characterized in that** the device is configured to perform the method according to claim 10.

12. The apparatus for transcranial brain stimulation according to one of claims 1 to 9, **characterized in that** the device for calibrating according to claim 11 is part of the apparatus.

## Revendications

1. Dispositif pour la stimulation transcrânienne du cerveau, comprenant :
- un ensemble d'électrodes constitué de plusieurs électrodes (2, 3) lesquelles, dans une configuration de fonctionnement, peuvent être disposées au niveau d'un crâne (4) d'un être humain ou d'un animal, dans lequel, dans la configuration de fonctionnement, en cas d'alimentation en courant des électrodes (2, 3), un flux de courant est généré à travers au moins un cortex auditif du cortex cérébral dans le crâne (4) ;
**caractérisé en ce que** le dispositif comprend :
- un dispositif de détection acoustique (1) pour la détection de signaux acoustiques (AS) en tant que signaux sonores analogiques (SI) ;
- un dispositif de conversion de signal (5) configuré pour,
i) convertir un signal sonore analogique (SI) respectif en un premier signal numérique (DS1), lequel correspond de par son allure au signal sonore analogique (SI) respectif, et déterminer à partir du premier signal numérique (DS1) un deuxième signal numérique (DS2) **en ce qu'**à partir du premier signal numérique (DS1), la courbe enveloppante (HK) est extraite et **en ce que** la courbe enveloppante (HK) est différée de l'ordre d'une quantité de temps (tl) prédéterminée supérieure à zéro ou bien reste non différée ;
ii) convertir le deuxième signal numérique (DS2) en un courant (I), lequel correspond de par son allure au deuxième signal numérique (DS2), et alimenter avec ce courant (I) l'ensemble d'électrodes dans la configuration de fonctionnement.

2. Dispositif selon la revendication 1, **caractérisé en ce que** l'ensemble d'électrodes se compose d'une paire d'électrodes (2, 3) pour le cortex auditif sur la moitié gauche du crâne et/ou d'une paire d'électrodes pour le cortex auditif dans la moitié droite du crâne.

3. Dispositif selon la revendication 1 ou 2, **caractérisé en ce que** la quantité de temps prédéterminée (tl) est comprise entre 50 ms et 150 ms, et est de préférence de l'ordre de 100 ms.

4. Dispositif selon l'une des revendications précédentes, **caractérisé en ce que** le dispositif de conversion de signal (5) est réalisé de manière à ce que, dans le cadre de l'extraction de la courbe enveloppante (HK), on réalise une transformation de Hilbert du premier signal numérique (DS1).

5. Dispositif selon la revendication 4, **caractérisé en ce que** le dispositif de conversion de signal (5) est réalisé de manière à ce que l'extraction de la courbe enveloppante (HK) comprend un filtrage passe-bande du premier signal numérique (DS1) après la transformation d'Hilbert de celui-ci dans une gamme de fréquences prédéfinie, dans lequel la gamme de fréquences prédéfinie est de préférence la bande comprise entre 0 et 10 Hz ou une sous-partie de cette bande.

6. Dispositif selon l'une des revendications précédentes, **caractérisé en ce que** le dispositif de conversion de signal (5) comprend une unité de traitement de signal (6, 7) et une unité de stimulation (8) qui sont réalisées de manière à ce que l'unité de traitement de signal (6, 7) réceptionne le signal sonore analogique (SI) respectif du dispositif de détection acoustique (I) et détermine à partir de cela le deuxième signal numérique (DS2), dans lequel le deuxième signal numérique (DS2) est tout d'abord converti par l'unité de traitement de signal (6, 7) en une tension (V) qui correspond de par son allure au deuxième signal numérique (DS2) et que l'on fait sortir à l'attention de l'unité de stimulation (8), laquelle convertit la tension (V) en courant (I) pour l'alimentation en courant de l'ensemble d'électrodes.

7. Dispositif selon l'une des revendications précédentes, **caractérisé en ce que** le courant (I) est défini de manière à ce que sa valeur maximale est de préférence comprise entre 0,5 mA et 5 mA, en étant en particulier de l'ordre de 2 mA ou moins et de préférence de 1 mA.

8. Dispositif selon l'une des revendications précédentes, **caractérisé en ce que** le dispositif peut être porté par l'être humain ou l'animal, au niveau du crâne (4) duquel les électrodes (2, 3) ou l'ensemble d'électrodes sont mises en place dans la configuration de fonctionnement, et peut en particulier être fixé au crâne (4) de l'être humain ou de l'animal.

9. Dispositif selon l'une des revendications précédentes, **caractérisé en ce que** le dispositif comprend un dispositif de sortie acoustique pour l'émission de signaux acoustiques sur la base des signaux sonores analogiques dans la zone d'une oreille, ou des deux d'entre-elles, de l'être humain ou de l'animal au niveau du crâne (4) duquel les électrodes (2, 3) ou l'ensemble d'électrodes sont mises en place dans la configuration de fonctionnement.

10. Procédé pour l'étalonnage d'un dispositif selon l'une des revendications précédentes, dans lequel
- en cas de présence d'un signal acoustique (AS), on détecte au moyen d'un ensemble d'électrodes constitué de plusieurs électrodes (2', 3') un électroencéphalogramme sous la forme d'une variation de tension dans le temps dans la zone d'au moins un cortex auditif du cortex cérébral dans le crâne (4) d'un être humain ou d'un animal pour lequel le dispositif pour la stimulation transcrânienne du cerveau est à utiliser;
- la courbe enveloppante (HK') de l'électroencéphalogramme est extraite et le décalage temporel avec la plus grand concordance entre la courbe enveloppante (HK) extraite par le dispositif pour la stimulation transcrânienne du cerveau, laquelle repose sur le signal acoustique (AS) présent, et la courbe enveloppante (HK') de l'électroencéphalogramme est déterminé au moyen d'une corrélation croisée ;
- le décalage temporel est déposé en tant que quantité de temps prédéterminée (tl) dans le dispositif pour la stimulation transcrânienne du cerveau.

11. Dispositif pour l'étalonnage d'un dispositif selon l'une des revendications 1 à 9, **caractérisé en ce que** le dispositif est configuré pour la mise en oeuvre du procédé selon la revendication 10.

12. Dispositif pour la stimulation transcrânienne du cerveau selon l'une des revendications 1 à 9, **caractérisé en ce que** le dispositif pour l'étalonnage selon la revendication 11 est un élément constitutif du dispositif.
